(19) Europäisches Patentamt
European Patent Office
Office européen des brevets

(11) Numéro de publication: **0 222 164**
**B1**

(12) # FASCICULE DE BREVET EUROPEEN

(45) Date de publication du fascicule du brevet: **31.05.89**

(51) Int. Cl.⁴: **C07D 307/60**, A23L 1/226

(21) Numéro de dépôt: **86113961.6**

(22) Date de dépôt: **08.10.86**

(54) **Esters furanniques et leur utilisation en tant qu'ingrédients aromatisants.**

(30) Priorité: **05.11.85 CH 4746/85**

(43) Date de publication de la demande:
**20.05.87 Bulletin 87/21**

(45) Mention de la délivrance du brevet:
**31.05.89 Bulletin 89/22**

(84) Etats contractants désignés:
**CH DE FR GB LI NL**

(56) Documents cités:
**FR-A- 2 118 954**
**US-A- 3 455 702**

**Le dossier contient des informations techniques présentées postérieurement au dépôt de la demande et ne figurant pas dans le présent fascicule.**


(73) Titulaire: **FIRMENICH SA, 1, route des Jeunes, CH-1211 Genève 8(CH)**

(72) Inventeur: **Pickenhagen, Wilhelm, Dr., Chemin du Mont-Mussy, CH-1290 Chavannes-des-Bois(CH)**
Inventeur: **Velluz, Alain, "Lanovaz", Arenthon, F-74800 La Roche/Foron(FR)**

(74) Mandataire: **Salvadori, Giuseppe, Dr., c/o Firmenich S.A. Case Postale 239, CH-1211 Genève 8(CH)**


**EP 0 222 164 B1**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

ACTORUM AG

**Description**

La présente invention a trait au domaine des arômes. Elle concerne en particulier l'utilisation de certains composés furanniques nouveaux en tant qu'ingrédients aromatisants destinés à renforcer, améliorer ou modifier les qualités aromatiques propres d'aliments ou boissons.

Les composés de l'invention obéissent à la formule générale (I) dans laquelle R sert à définir un reste alkyle $C_2$–$C_6$ saturé linéaire ou ramifié. Il s'agit donc d'esters furanniques de la famille de la 4-hydroxy-2,5-diméthyl-2,3-dihydrofuran-3-one, mieux connue dans l'art sous la dénomination de FURANEOL (marque enregistrée de Firmenich SA, Genève). Depuis sa découverte, ce composé s'est imposé comme un ingrédient critique non seulement pour toute reconstitution de l'arôme de fraise, mais également en tant que constituant d'arômes de fruits variés, voire d'arômes viandeux.

Nombreuses ont été les études consacrées à ce composé; mais, fait étonnant, aucune attention particulière n'a été vouée à l'examen des propriétés organoleptiques de ses dérivés esters.

Le brevet suisse No 486 850 (publié le 30. 4. 1970) mentionne que certains dérivés pouvant se dégrader in situ pour fournir du Furanéol dans des aliments ou boissons peuvent être employés en remplacement de celui-ci. Le 2,5-diméthyl-3-acétoxy-4-oxo-4,5-dihydrofuranne est mentionné à ce titre.

La DE-OS 2 359 891 (publiée le 6. 6. 1974) décrit certains esters furanniques de formule (II) dans laquelle R représente un reste alkyle $C_1$–$C_5$, $R^2$ représente un groupe alkyle $C_1$–$C_4$ linéaire ou ramifié et $R^3$ sert à désigner un atome d'hydrogène ou un groupe alkyle $C_1$–$C_7$, en tant qu'intermédiaires dans un procédé de synthèse de furanones.

La demande FR-A 2 118 954 décrit un procédé de préparation de certains dérivés furanniques, en particulier de certains 2,3-dihydro-furanne-3-ones substitués. Il y est fait mention que les composés ainsi préparés sont des agents aromatisants ou précurseurs d'agents aromatisants importants sans toutefois définir la nature exacte de ces composés ni distinguer entre ceux des composés ayant une action aromatisante et ceux pouvant servir en tant que précurseurs des composés actifs.

Aucune mention n'a été faite dans l'art antérieur quant aux qualités aromatisantes des esters de formule (I).

Nous avons maintenant découvert que lesdits esters possédaient des propriétés fort intéressantes et que de ce fait ils pouvaient être employés avantageusement pour l'aromatisation d'aliments variés, de boissons et du tabac.

Notre invention est basée sur la découverte fortuite que le butanoate de 2,5-diméthyl-4-oxo-3(5H)-furyle était un constituant de l'absolue obtenue à partir d'un extrait de fraises lyophilisées dans lequel il est présent à une concentration inférieure à environ 70 ppm. Il s'agit donc d'un composé d'origine naturelle dont la présence dans l'arôme de fraise avait échappée jusqu'ici aux nombreux examens et analyses auxquels avaient été soumis les différents extraits obtenus des fruits d'origine variée.

L'extrait a été obtenu par un procédé en plusieurs étapes qui a consisté en la lyophilisation de fraises fraîches, la congélation dans l'azote liquide et l'extraction à l'éther diéthylique de la poudre obtenue par broyage des fruits lyophilisés congelés. Après concentration, l'extrait éthéré a été repris à l'éthanol et maintenu sous agitation à 40°C pendant 30 minutes. Après refroidissement à –7°C et filtration à froid, l'alcool a été éliminé par distillation. Le résidu ainsi obtenu a été soumis à une séparation chromatographique sur colonne en utilisant comme support du gel de silice et comme éluant du pentane et de l'éther diéthylique. 35 fractions ont été ainsi obtenues, parmi lesquelles trois seulement contenaient l'ester en question dont l'identité a été confirmée par une comparaison avec un échantillon obtenu par esterification directe du Furanéol avec l'anhydride butyrique.

Quoique les propriétés aromatisantes du butanoate de 2,5-diméthyl-4-oxo-3(5H)-furyle s'apparentent à celles du Furanéol, notamment pour sa note caramel, elles diffèrent du composé connu par son intensité, son caractère plus beurré et par une note moins fruitée. De par ces caractères, et par sa puissance, l'ester mentionné peut donc constituer, suivant le type d'application, un ingrédient d'emploi alternatif ou complémentaire à celui du Furanéol.

Par ailleurs, les composés définis à l'aide de la formule (I), quoique caractérisés par des légères différences structurales, montrent à l'expérience des différences plus ou moins prononcées quant à l'intensité de leur arôme et à leur qualité.

Le tableau en annexe résume les propriétés de différents esters selon l'invention en regard de leur formule spécifique.

Parmi les composés de formule (I), il s'est avéré que le produit qui possédait au mieux les caractères utiles propres à l'emploi envisagé était le butanoate de 2,5-diméthyl-4-oxo-3(5H)-furyle. Toutefois, les autres dérivés analogues définis par la formule (I) peuvent trouver un emploi particulier suivant l'effet aromatisant recherché et la nature du produit que l'on désire aromatiser.

Les qualités des esters de l'invention apparaissent comme tout à fait surprenantes au vu des connaissances acquises dans l'art.

L'homme de l'art sait par expérience que des légères modifications moléculaires s'accompagnent dans la plupart des cas de modifications souvent fondamentales des propriétés organoleptiques, et ce sans raison apparente.

Les proportions dans lesquelles les esters de l'invention peuvent être employés pour produire les effets recherchés varient, comme souvent en pareil cas, dans une gamme de valeurs assez étendue. Leur

valeur dépendra en premier lieu de l'effet spécifique désiré, de la nature du produit que l'on désire aromatiser et de la nature des coingrédients dans une composition aromatisante donnée. Généralement ces proportions sont de l'ordre de 1 à 100 parties par millions, de préférence comprises entre 5 et 20 ppm.

Les esters de l'invention sont incorporés aux aliments, boissons, préparations pharmaceutiques ou tabac que l'on désire aromatiser selon des procédés usuels dans l'art, généralement en mélange avec d'autres ingrédients aromatisants naturels ou synthétiques. Leur emploi a lieu de préférence en solution dans l'un des solvants comestibles usuels tels la triacétine, l'alcool éthylique ou le propylène glycol, ou en mélange sur un support solide, par exemple une dextrine ou la gomme arabique.

A titre d'aliment pouvant être aromatisé on peut mentionner en tant qu'exemple les glaces, les crèmes à dessert, les yogourths, les produits lactés en général, les produits de confiserie ou de boulangerie, les sirops, les sucres cuits ou les confitures, ou encore les bouillons, les extraits concentrés solides pour la préparation de soupes et des sauces ou en général les produits à base de viande ou imitant la viande.

Le terme "tabac" tel qu'utilisé ici, doit être interprété comme comprenant des produits naturels, tel le tabac burley, le tabac turc ou de Maryland, ou des tabacs reconstitués ou homogénéisés, ainsi que des produits artificiels de remplacement du tabac. Les articles préparés à l'aide desdits produits de tabac peuvent tout aussi bien être des articles à fumer, que des articles à mâcher ou à priser.

Les esters de l'invention peuvent être aisément obtenus par un procédé simple d'esterification à partir de Furanéol. En fonction de l'ester désiré l'on utilisera l'anhydride ou l'halogénure de l'acide carboxylique approprié suivant le schéma (A).

Le procédé suivi est en soi courant et la méthode spécifique employée sera illustrée plus en détail par les exemples donnés ci-après. Dans la section suivante seront également décrites certaines applications particulières, mais bien entendu l'invention ne saurait être limitée aux exemples donnés. Les températures sont indiquées en degrés centigrades et les abréviations ont le sens usuel dans l'art.

Exemple 1

Préparation du butanoate de 2,5-diméthyl-4-oxo-3(5H)-furyle

158 Grammes (1 M) d'anhydride butyrique ont été introduits à 0–3° goutte à goutte, sous azote et avec agitation dans un mélange de 128 g (1 M) de Furanéol (produit commercial de Firmenich SA) et 385 g (4,9 M) de pyridine.

Le mélange de réaction a été laissé sous agitation pendant que la température remonte à la valeur ambiante. Le mélange a été ensuite extrait à l'éther (100 ml), puis l'extrait éthéré a été soumis aux traitements usuels de neutralisation avec deux fractions de 25 ml de NaOH aqueux à 10%, lavage avec de l'eau saturée au NaCl et séchage sur $MgSO_4$. L'évaporation sous vide du solvant a fourni un résidu qui par distillation sur une colonne Vigreux a fourni 123 g d'un produit ayant Eb. 70°/11,6 Pa.

Exemple 2

Préparation du 2-méthyl-butanoate de 2,5-diméthyl-4-oxo-3(5H)-furyle

5,28 Grammes (0,044 M) de chlorure de 2-méthyl-butanoyle ont été introduits goutte à goutte à 0–3°, sous azote et avec agitation, dans un mélange de 5,12 g (0,04 M) de Furanéol et de 12,8 ml (0,2 M) de pyridine. Après avoir laissé remonter la température à la valeur ambiante, le mélange a été traité avec 50 ml d'éther. Comme à l'exemple 1, l'extrait éthéré a été soumis aux traitements usuels. Le résidu obtenu par évaporation du solvant a été distillé sous vide dans un four à boules pour fournir l'ester désiré ayant Eb. 87°/12 Pa.

En suivant l'une des méthodes générales indiquées ci-dessus, on a préparé les esters figurant dans le tableau suivant. En regard de chaque composé figurent les données analytiques (IR, RMN et SM) correspondantes.

a. 2-Méthylbutanoate de 2,5-diméthyl-4-oxo-3(5H)-furyle ($C_{11}H_{16}O_4$)
RMN ($CDCl_3$): 1,0 (3H, t); 1,26 (3H, d); 1,49 (3H, d); 1,6 (1H, m); 1,79 (1H, m); 2,15 (3H, s); 2,62 (1H, m); 4,57 (1H, q) delta ppm;
IR ($CHCl_3$): 2960, 2930, 2870, 1755, 1705, 1630, 1300, 1185, 1135, 1100, 995 $cm^{-1}$;
SM: $M^+$ = 212 (8); m/e: 41 (11), 43 (28), 57 (100), 72 (10), 85 (73), 128 (38), 129 (16).

b. 3-Méthylbutanoate de 2,5-diméthyl-4-oxo-3(5H)-furyle ($C_{11}H_{16}O_4$)
RMN ($CDCl_3$): 1,04 (6H, d); 1,5 (3H, d); 2,16 (3H, s); 2,19 (1H, m); 2,41 (2H, d); 4,56 (1H, q) delta ppm;
IR ($CHCl_3$): 2990, 2955, 2900, 1770, 1720, 1645, 1315, 1195, 1150, 1100, 1010 $cm^{-1}$;
SM: $M^+$ = 212 (11); m/e: 39 (6), 41 (22), 43 (47), 57 (100), 72 (9), 85 (87), 128 (75), 129 (11).

c. Butanoate de 2,5-diméthyl-4-oxo-3(5H)-furyle ($C_{10}H_{14}O_4$)
RMN ($CDCl_3$): 1,01 (3H, t); 1,49 (3H, d); 1,74 (2H, m); 2,16 (3H, s); 2,52 (2H, t); 4,56 (1H, q) delta ppm;
IR($CHCl_3$): 2960, 2930, 2875, 1760, 1710, 1630, 1305, 1185, 1135, 1095, 995 $cm^{-1}$;
SM: $M^+$ = 198 (7); m/e: 41 (9), 43 (100), 57 (11), 71 (51), 72 (11), 85 (33), 128 (54).

d. 2-Buténoate de 2,5-diméthyl-4-oxo-3(5H)-furyle ($C_{10}H_{12}O_4$)
RMN ($CDCl_3$): 1,51 (3H, d); 1,75 (3H, d); 2,17 (3H, s); 4,58 (1H, q); 6,01 (1H, d); 7,18 (1H, dxq) delta ppm;

IR ($CHCl_3$): 2990, 2925, 1735, 1700, 1625, 1300, 1285, 1185, 1145, 1095, 995, 960 $cm^{-1}$;
SM: $M^+$ = 196 (4); m/e: 41 (16), 43 (9), 69 (100), 128 (9).

e. Pentanoate de 2,5-diméthyl-4-oxo-3(5H)-furyle ($C_{11}H_{16}O_4$)
RMN ($CDCl_3$): 0,95 (3H, t); 1,43 (2H, m); 1,5 (3H, d); 1,7 (2H, m); 2,16 (3H, s); 2,54 (2H, t); 4,57 (1H, q) delta ppm;
IR ($CHCl_3$): 2955, 2930, 2875, 1760, 1705, 1630, 1415, 1300, 1185, 1135, 995 $cm^{-1}$;
SM: $M^+$ = 212 (4); m/e: 41 (19), 43 (31), 55 (14), 57 (100), 72 (11), 85 (93), 128 (66).

f. Hexanoate de 2,5-diméthyl-4-oxo-3(5H)-furyle ($C_{12}H_{18}O_4$)
RMN ($CDCl_3$): 0,92 (3H, t); 1,36 (4H, m); 1,5 (3H, d); 1,72 (2H, m); 2,16 (3H, s); 2,53 (2H, t); 4,57 (1H, q) delta ppm;
IR ($CHCl_3$): 2955, 2925, 2870, 1760, 1705, 1630, 1300, 1185, 1135, 1095, 995 $cm^{-1}$;
SM: $M^+$ = 226 (3); m/e: 41 (11), 43 (100), 55 (17), 57 (10), 71 (69), 72 (15), 85 (35), 99 (51), 128 (76), 129 (11).

Exemple 3

Comparaison aromatique

On a procédé à une comparaison des qualités aromatiques du butanoate de 2,5-diméthyl-4-oxo-3(5H)-furyle avec celles développées par le Furanéol en examinant un échantillon des produits en question à un dosage de 20 ppm dans de l'eau de source. Les commentaires exprimés par un panel d'expert sont reproduits ci-après:

Furanéol: typique caramel, fruité, légèrement brûlé, sucré, fraise

Butanoate de 2,5-diméthyl-4-oxo-3(5H)-furyle: caramel, moins fruité que le Furanéol, plus beurré, plus puissant

Une comparaison analogue a été effectuée sur un échantillon d'autres esters de l'invention et le résultat observé a été reproduit dans la section précédente de la spécification.

Exemple 4

Composition aromatisante fruitée

Une composition aromatisante de base de type fruité ananas a été préparée en mélangeant les ingrédients suivants (parties en poids):

| | |
|---|---|
| Vanilline | 3,0 |
| Extrait conc. d'essence d'orange | 5,0 |
| Extrait conc. d'essence de citron | 10,0 |
| Butyrate d'éthyle | 10,0 |
| Hexanoate d'éthyle | 0,5 |
| Hexanoate d'allyle | 12,0 |
| Isovalérianate d'éthyle | 3,0 |
| Acétate d'isoamyle | 0,5 |
| alpha-Ionone à 10%* | 0,5 |
| Oenanthate d'éthyle | 1,0 |
| Ethanol à 95% (v/v) | 54,5 |
| | 100,0 |

* dans l'éthanol à 95%

En utilisant la base indiquée ci-dessus, on a préparé deux compositions ainsi:

| | Arôme A | Arôme B |
|---|---|---|
| Butanoate de 2,5-diméthyl-4-oxo-3(5H)-furyle | – | 0,5 |
| Base ananas | 10,0 | 10,0 |
| Ethanol à 95% | 90,0 | 89,5 |
| | 100,0 | 100,0 |

Les deux compositions résultantes ont été évaluées dans une solution sucrée acide (10% de saccharose et 0,10% d'acide citrique dans l'eau de source) à raison de 0,10%.

Le groupe d'experts appelés à se prononcer sur les qualités des deux arômes obtenus a trouvé que l'arôme B était plus complet, plus doux, harmonieux et naturel que l'arôme A; il possédait en outre un caractère ananas plus caractéristique.

Exemple 5

Composition aromatisante fruitée

Une composition aromatisante de base de type fruité fraise a été préparée en mélangeant les ingrédients suivants (parties en poids).

| | |
|---|---|
| Butyrate d'éthyle | 3,5 |
| Méthylphénylglycidate d'éthyle | 0,7 |
| cis-3-Hexénol | 0,5 |
| Acétate d'isobutyle à 10%* | 3,5 |
| Acétate de styrallyle à 10%* | 1,5 |
| 2-Méthylbutyrate d'éthyle à 10%* | 1,0 |
| Gamma-décalactone | 0,5 |
| Cinnamate de méthyle à 10%* | 10,0 |
| Ethanol à 95% (v/v) | 78,8 |
| | 100,0 |

* dans l'éthanol à 95%

En utilisant la base indiquée ci-dessus on a préparé deux compositions ainsi:

| | Arôme A | Arôme B |
|---|---|---|
| Butanoate de 2,5-diméthyl-4-oxo-3(5H)-furyle | – | 0,7 |
| Base fraise | 10,0 | 10,0 |
| Ethanol à 95% | 90,0 | 89,3 |
| | 100,0 | 100,0 |

Les deux compositions résultantes ont été évaluées dans une solution sucrée acide (10% de saccharose et 0,10% d'acide citrique dans l'eau de source) à raison de 0,10%.

De l'avis d'un groupe d'experts, l'arôme B était plus mûre, fruité et légèrement plus beurré que l'arôme A; il possédait en outre un caractère fraise plus prononcé. L'arôme A montrait par contre une note plus verte.

Exemple 6

Composition aromatisante de type beurre

Une composition aromatisante de type beurre a été préparée en mélangeant les ingrédients suivants (parties en poids):

| | |
|---|---|
| Vanilline | 1,5 |
| Diacétyle | 3,0 |
| Acétylméthylcarbinol | 10,0 |
| Acide butyrique | 15,0 |
| Gamma-octalactone 10%* | 2,5 |
| Butyrate d'éthyle | 10,0 |
| Gamma undécalactone 1%* | 10,0 |
| Huile végétale[1)] | 48,0 |
| | 100,0 |

* dans l'éthanol à 95%
1) fraction de l'huile de coco

A l'aide de ladite composition de base, on a préparé deux autres compositions en mélangeant les ingrédients suivants:

| | Arôme A | Arôme B |
|---|---|---|
| Butanoate de 2,5-diméthyl-4-oxo-3(5H)-furyle | – | 0,5 |
| Base beurre | 10,0 | 10,0 |
| Huile végétale (vide supra) | 90,0 | 89,5 |
| | 100,0 | 100,0 |

On a ensuite préparé des biscuits à pâte tendre de type anglais en mélangeant les ingrédients suivants (parties en poids):

| | | |
|---|---|---|
| Sucre | 210 | X |
| Margarine | 175 | X |
| Sirop de sucre (65%) | 40 | X |
| Poudre de lait | 30 | X |
| Œufs en poudre | 20 | X |
| Arôme (A ou B) | 38 | |
| Farine | 600 | |
| Sel | 6 | |
| Bicarbonate d'ammonium | 7 | |
| Bicarbonate de sodium | 6 | |
| Eau | 180 | |
| | 1312 | |

L'arôme a été ajouté sous forme de prémélange dans le sirop de sucre au mélange X constitué par la matière grasse, le sirop et le sucre afin de garantir sa bonne diffusion dans la pâte.

Le mélange ainsi obtenu contenant l'arôme a été brassé pendant 3 minutes dans un appareil approprié, puis les autres ingrédients y ont été ajoutés tout en mélangeant pendant 3 autres minutes.

La pâte tendre a été pressée sur rouleau et déposée sur plaques à une épaisseur de 3 mm. Les biscuits ont été saupoudrés légèrement avec du sucre avant leur cuisson. Celle-ci s'effectue dans un four rotatif de laboratoire à une température de 210°, la première minute avec de la vapeur.

De l'avis du groupe d'experts appelés à se prononcer sur les qualités organoleptiques des biscuits ainsi obtenus, ceux qui avaient été aromatisés à l'aide de l'arôme B possédaient un goût et un arôme plus riches et plus complets que ceux qui avaient été aromatisés à l'aide de l'arôme A; ils montraient en outre un caractère "cuit" plus prononcé.

Exemple 7

Aromatisation d'un produit viandeux

Une reconstitution d'un bouillon de bœuf a été préparée en mélangeant les ingrédients suivants (parties en poids):

| | |
|---|---|
| Extrait de bœuf commercial | 10 |
| Glutamate monosodique | 1 |
| Mélange 50:50 de sodium inositate et sodium guanilate | 0,005 |
| Chlorure de sodium | 8 |
| Acide lactique | 0,5 |
| Eau | 980,495 |
| Total | 1000,0 |

Le bouillon ainsi obtenu a été ensuite réparti en deux portions de volume égale et à l'une d'entre elles on a ajouté le butanoate de 2,5-diméthyl-4-oxo-3(5H)-furyle à raison de 2 ppm. La fraction ainsi aromatisée a été finalement soumise à évaluation par comparaison avec la fraction non aromatisée.

De l'avis d'un panel, le bouillon aromatisé possède un arôme plus riche et plus complet avec une tonalité légèrement plus rôtie et juteuse que le bouillon non aromatisé.

Annexe

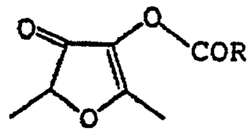

(I)

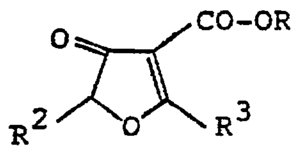

(II)

Schéma (A)

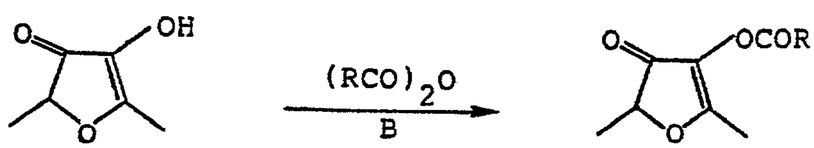


(I)

B = base

Annexe

Tableau

| | Structure | Qualités aromatiques |
|---|---|---|
| a. | 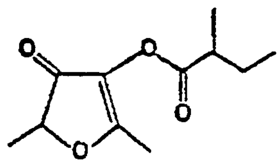 | gras, plastique, légèrement fromage (cheesy) |
| b. | 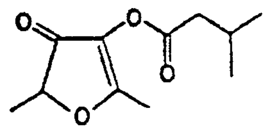 | beurré, anisique |
| c. | 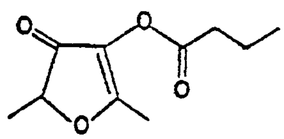 | caramel, fraise, moins fruité et plus beurré que le Furanéol |
| d. | 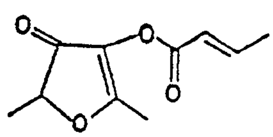 | métallique, moins fruité que c. |
| e. | 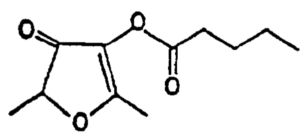 | fruité, plus brûlé que c. |
| f. | 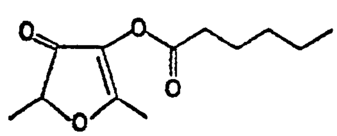 | gras, brûlé, fruité, fraise |

a. 2-Méthylbutanoate de 2,5-diméthyl-4-oxo-3(5H)-furyle
b. 3-Méthylbutanoate de 2,5-diméthyl-4-oxo-3(5H)-furyle
c. Butanoate de 2,5-diméthyl-4-oxo-3(5H)-furyle
d. 2-Buténoate de 2,5-diméthyl-4-oxo-3(5H)-furyle
e. Pentanoate de 2,5-diméthyl-4-oxo-3(5H)-furyle
f. Hexanoate de 2,5-diméthyl-4-oxo-3(5H)-furyle

**Revendications**

1. Composés de formule (I)

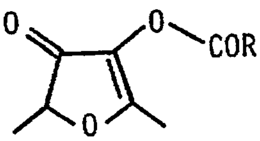

(I)

dans laquelle R sert à définir un reste alkyle $C_2$–$C_6$ saturé linéaire ou ramifié.

2. A titre de composé selon la revendication 1, le butanoate de 2,5-diméthyl-4-oxo-3(5H)-furyle.

3. Utilisation d'un composé selon la revendication 1 en tant qu'ingrédient aromatisant.

4. Composition aromatisante résultant de l'utilisation selon la revendication 3.

5. Aliment, boisson, préparation pharmaceutique ou tabac contenant à titre d'ingrédient aromatisant une quantité efficace d'un des composés selon la revendication 1.

**Claims**

1. Compounds of formula

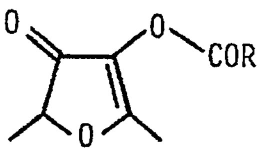

(I)

wherein R represents a $C_2$–$C_6$ linear or branched saturated alkyl radical.

2. As a compound according to claim 1, 2,5-dimethyl-4-oxo-3(5H)-furyl butanoate.

3. Utilization of a compound according to claim 1 as flavouring ingredient.

4. Flavouring composition resulting from the utilization according to claim 3.

5. A foodstuff, a beverage, a pharmaceutical preparation or tobacco containing as a flavouring ingredient an effective quantity of one of the compounds according to claim 1.

**Patentansprüche**

1. Verbindungen der Formel

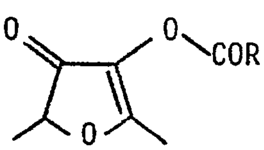

(I)

worin R einen $C_2$ bis $C_6$ linearen oder verzweigten gesättigten Alkylrest darstellt.

2. Als Verbindung gemäß Anspruch 1, 2,5-dimethyl-4-oxo-3(5H)-furyl butanoate.

3. Verwendung einer Verbindung gemäß Anspruch 1 als Geschmackstoffzusatz.

4. Aromazusammensetzung resultierend aus der Verwendung gemäß Anspruch 3.

5. Ein Nahrungsmittel, ein Getränk, ein pharmazeutisches Präparat oder Tabak, die als Geschmackstoffzusatz eine wirksame Menge einer Verbindung gemäß Anspruch 1 enthalten.